# EUROPEAN PATENT APPLICATION

(11) **EP 4 712 094 A1**
(43) Date of publication of application: **18.03.2026**
(21) Application number: 25201011.1
(22) Date of filing: 09.09.2025
(51) Int. Cl.: G16H 20/30, G16H 40/63, G16H 40/67, G16H 50/20, G16H 50/70, A63B 24/00

(54) **METHOD FOR MONITORING AND RECONSTRUCTING HEART RATE VALUES OF A USER OF A GYMNASTIC MACHINE AND GYMNASTIC MACHINE IMPLEMENTING THE METHOD**

(30) Priority: 13.09.2024 IT 202400020413
(71) Applicant: Technogym S.p.A., 47521 Cesena (FC) (IT)
(72) Inventor: PASINI, Alessandro, 47521 Cesena (FC) (IT)
(74) Representative: Botti & Ferrari S.p.A.

(57) **Abstract**

Method (100) for monitoring and reconstructing heart rate values of a user of a gymnastic machine (M), of the type comprising at least one contact portion (3) with the user, an interface unit (4) operatively connected to at least one control logic unit (U, C), where the user heart rate values are stored, comprising the following steps:
110. acquiring heart rate values by means of said at least one contact portion (3) with the user while performing the workout;
120. sending said heart rate values to said at least one control logic unit (U, C);
130. displaying the heart rate signal trend and/or the current heart rate numerical values on said interface unit (4);
140. reconstructing the missing portions of the heart rate signal trend and/or missing current heart rate numerical values by means of said heart rate signal trends and/or stored previous heart rate numerical values, when the user is not in contact with said at least one contact portion (3);
150. displaying the heart rate signal trend and/or the reconstructed current heart rate numerical values on said interface unit (4) during the workout.

## Description

### Field of application

The present invention relates to a method for monitoring and reconstructing heart rate values of a user performing a workout on a gymnastic machine, being in contact with at least one portion of the gymnastic machine that detects heart values.

The present invention also relates to a machine that implements the method.

More specifically, the invention concerns a method of the above mentioned type, specifically designed and made to monitor heart rate values of a user performing a workout on a gymnastic machine, being in contact with at least one portion of the gymnastic machine able to detect heart rate values, and to reconstruct the heart rate signal trend or the current numerical values when the user is not in contact with the portion of the machine during the workout.

The following description will be directed to a method and a machine for monitoring and reconstructing heart rate values of a user performing a workout on a gymnastic machine of the treadmill type provided with heart rate sensors or hand sensors, but it is well clear that this should not be considered limited to this specific use.

### Prior art

It is currently known that gymnastic machines, in particular treadmills, allow a user performing a workout to display his/her heart rate trend, as well as other parameters relating to the workout, on a graphical interface.

Usually, heart rate is detected by means of a contact portion, comprising sensors, arranged on the frame of the gymnastic machine, on which the user places his/her hands.

However, during the workout, it is possible for the user to remove his/her hands from the contact portion, causing the interruption of the heart rate acquisition.

As a result, a heart rate trend is displayed on the graphical interface with interruptions at the time intervals when the user's hands are not in contact with the contact portions.

In light of the prior art drawbacks and unsolved problems, the object of the present invention is to provide a method for monitoring heart rate values.

A further object of the present invention is to reconstruct the heart rate signal trend and/or the current heart rate numerical values and display them on the user interface, even when the user is not in contact with the machine.

A further object of the present invention is to provide a machine for performing the method.

Therefore, the technical problem underlying the present invention is to provide a method and a machine for monitoring and reconstructing heart rate values and displaying them on a user interface, even when the user is not in contact with the machine.

### Summary of the invention

The idea underlying the present invention is to provide a method for monitoring and reconstructing heart rate values of a user of a gymnastic machine and a system that implements the method.

The object of the present invention is a method for monitoring and reconstructing heart rate values of a user of a gymnastic machine, of the type comprising at least one contact portion with the user, an interface unit operatively connected to at least one control logic unit, where the user's heart rate values are stored, comprising the following steps:
110. acquiring heart rate values by means of said at least one contact portion in contact with the user while performing the workout;
120. sending said heart rate values to said at least one control logic unit;
130. displaying the heart rate signal trend and/or the current heart rate numerical values on said interface unit;
140. reconstructing the missing portions of the heart rate signal trend and/or the missing current heart rate numerical values by means of said heart rate signal trends and/or stored previous heart rate numerical values, when the user is not in contact with said at least one contact portion;
150. displaying the heart rate signal trend and/or the reconstructed current heart rate numerical values on said interface unit during the workout.

Preferably according to the invention, said step 140. is performed by an Artificial Intelligence module - AI.

Furthermore, according to the invention, said Artificial Intelligence module - AI is trained with heart rate signal trends and/or heart rate numerical values from the user's previous workouts, associated with the operating data of the gymnastic machine.

Still according to the invention, said AI module is trained with the heart rate values of a plurality of users, divided into clusters or groups of pre-determined features, such as age, gender, weight, and height.

A further object of the present invention is a gymnastic machine comprising at least one contact portion with the user, an interface unit operatively connected to at least one control logic unit, where heart rate signal trends and/or previous heart rate numerical values of the user are stored, being able to acquire heart rate values by means of said at least one contact portion with the user while performing the workout, send said heart rate values to said control logic unit, display the heart rate signal trend and/or the current heart rate numerical values on said interface unit, reconstruct the heart rate signal trend and/or the missing current heart rate numerical values by means of said stored trends and/or previous numerical values, when the user is not in contact with said at least one contact portion, display the heart rate signal trend and/or the reconstructed current heart rate numerical values on said interface unit during the workout.

Preferably according to the invention, said control logic unit comprises an Artificial Intelligence module - AI able to interpolate the missing heart rate values.

Furthermore, according to the invention, said control logic unit is comprised in said gymnastic machine.

Still according to the invention, said control logic unit is comprised in a remote cloud unit.

Furthermore, according to the invention, said machine may comprise a first control logic unit, operatively connected to a remote cloud unit.

Preferably, according to the invention, said machine may comprise a storage unit, where the user's heart rate values detected in previous workout sessions are stored, and said control logic unit may be operatively connected to said storage unit.

### Brief description of the drawings

In the drawings:
- figure 1 shows a perspective view of the gymnastic machine, object of the present invention; and

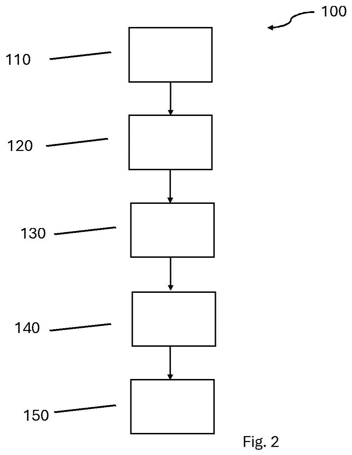

- figure 2 shows a block diagram of the operation of the monitoring and reconstructing method, object of the present invention.

### Detailed description

With reference to the appended figures, the method 100 for monitoring and reconstructing the heart rate values of a user of a gymnastic machine M, object of the present invention, allows monitoring and displaying the user's heart rate values on an interface unit 4 of the gymnastic machine M during a workout in which the user is in contact with a contact portion 3 of the gymnastic machine M, comprising heart rate sensors, and reconstructing and displaying the heart rate signal trend and/or the current heart rate numerical values.

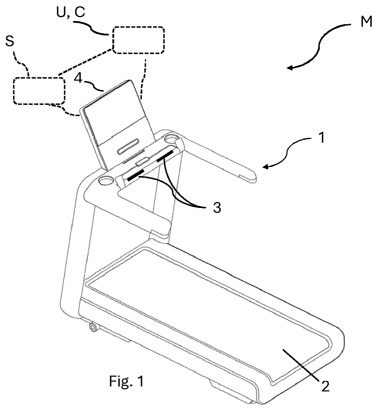

With reference to figure 1, the gymnastic machine M is a treadmill comprising a frame 1 and an exercise surface 2 on which the user can perform the workout.

On the frame 1 there is a contact portion 3 comprising heart rate sensors for detecting the heart rate values of the user who places his/her hands on the contact portion 3 during the workout.

In particular, the contact portion 3 comprises a portion for the right hand and a portion for the left hand.

The gymnastic machine M includes an interface unit 4, such as a display, on which the user can display the trend of his/her heart rate and other parameters and/or data during the workout.

Said gymnastic machine M also includes a control logic unit U for the operation of the machine M itself.

The user's heart rate values detected in previous workout sessions are stored in the control logic unit U.

An Artificial Intelligence module - AI able to reconstruct the user's heart rate signal trend and/or the current heart rate numerical values, even in the absence of detected values, by interpolating the detected heart rate values, is stored in the control logic unit U.

Without departing from the scope of protection of the present invention, said gymnastic machine M comprises a control logic unit U and a memory unit S operatively connected to said control logic unit U, where the user's heart rate values detected in previous workout sessions are stored.

In particular, said AI module is trained with the user's heart rate values for each workout session performed by the user. Furthermore, said Al module is also trained with the heart rate values of a plurality of users, divided into clusters or groups of pre-determined features, such as age, gender, weight, height and the like. In particular, a user's heart rate value is associated with each sliding speed and each slope of the exercise surface 2. Said AI module is therefore able to assign a heart rate value even at times when the user does not place his/her hands on the contact portion 3 of the machine during the workout.

Said gymnastic machine M may also be operatively connected to a remote control logic unit U, such as a cloud unit C, where the user's previous heart rate reference values and the Artificial Intelligence module AI are stored. The cloud C is operatively connected to the control logic unit U of the gymnastic machine M.

In operation, the machine M implements the following method.

In a step 110, the user's heart rate values are acquired during the workout by means of the contact portion 3.

In a step 120, the acquired values are sent to the control logic unit U or to the cloud C.

In a step 130, the heart rate signal trend and/or the acquired current heart rate numerical values are displayed on the interface unit 4.

In a step 140, the Artificial Intelligence module interpolates the heart rate signal trend and/or the current heart rate numerical values by means of the previous values and the stored data when the user is not in contact with the contact portion 3.

In a step 150, the complete trend of the interpolated heart rate signal is displayed on the interface unit 4 during the workout and/or the current heart rate numerical values.

Therefore, the user is always updated on the trend of his/her heart rate values during the workout, even if at certain times he/she cannot place his/her hands in contact with the contact portion 3.

As it is clear from the above description, the advantage of the method and machine object of the present invention is to acquire, interpolate, and display the heart rate signal trend and/or the current heart rate numerical values of a user during a workout on a gymnastic machine, even when he/she is not in contact with the gymnastic machine itself.

Obviously, a person skilled in the art, in order to meet contingent and specific needs, can make several changes and variants to the support described above, all of them by the way contained in the scope of protection of the invention as defined by the following claims.

## Claims

1. Method (100) for monitoring and reconstructing heart rate values of a user of a gymnastic machine (M), of the type comprising at least one contact portion (3) with the user, an interface unit (4) operatively connected to at least one control logic unit (U, C), where the user heart rate values are stored, comprising the following steps:
110. acquiring heart rate values by means of said at least one contact portion (3) with the user while performing the workout;
120. sending said heart rate values to said at least one control logic unit (U, C);
130. displaying the heart rate signal trend and/or the current heart rate numerical values on said interface unit (4);
140. reconstructing the missing portions of the heart rate signal trend and/or missing current heart rate numerical values by means of said heart rate signal trends and/or stored previous heart rate numerical values, when the user is not in contact with said at least one contact portion (3);
150. displaying the heart rate signal trend and/or reconstructed current heart rate numerical values on said interface unit (4) during the workout.

2. Method (100) according to the preceding claim, **characterized in that** said step 140. is performed by an Artificial Intelligence module - AI .

3. Method (100) according to the preceding claim, **characterized in that** said Artificial Intelligence module - AI is trained with heart rate signal trends and/or heart rate numerical values from the user's previous workout sessions, associated with the operating data of the gymnastic machine (M).

4. Method (100) according to any one of claims 2 or 3, **characterized in that** said AI module is trained with the heart rate values of a plurality of users, divided into clusters or groups of pre-determined features, such as age, gender, weight, and height.

5. Gymnastic machine (M) comprising at least one contact portion (3) with the user, an interface unit (4) operatively connected to at least one control logic unit (U, C), where the heart rate signal trends and/or previous heart rate numerical values of the user are stored, being able to acquire heart rate values by means of said at least one contact portion (3) with the user while performing the workout, send said heart rate values to said control logic unit (U, C), display the heart rate signal trend and/or the current heart rate numerical values on said interface unit (4), reconstruct the heart rate signal trend and/or the missing current heart rate numerical values by means of said stored trends and/or previous numerical values, when the user is not in contact with said at least one contact portion (3), display the heart rate signal trend and/or the reconstructed current heart rate numerical values, on said interface unit (4) during the workout.

6. Machine (M) according to the preceding claim, **characterized in that** said control logic unit (U, C) comprises an Artificial Intelligence module - AI, able to interpolate the missing heart rate values.

7. Machine (M) according to any one of claims 5 or 6, **characterized in that** said control logic unit (U) is comprised in said gymnastic machine (M).

8. Machine (M) according to any one of claims 5 or 6, **characterized in that** said control logic unit (C) is comprised in a remote cloud unit.

9. Machine (M) according to any one of claims 5 or 6, **characterized by** comprising a first control logic unit (U), operatively connected to a remote cloud unit (C).

10. Machine (M) according to any one of claims 5-9, **characterized by** comprising a storage unit (S), where the user's heart rate values in previous workout sessions are stored; and
in that said control logic unit (U) is operatively connected to said storage unit (S).
